(19) Europäisches Patentamt
European Patent Office
Office européen des brevets

(11) **EP 4 074 817 B1**

(12) **EUROPEAN PATENT SPECIFICATION**

(45) Date of publication and mention
of the grant of the patent:
**08.04.2026 Bulletin 2026/15**

(21) Application number: **20899125.7**

(22) Date of filing: **13.07.2020**

(51) International Patent Classification (IPC):
*C12N 1/20* $^{(2026.01)}$  *C12N 1/02* $^{(2006.01)}$
*A23L 33/135* $^{(2016.01)}$  *A23C 9/123* $^{(2006.01)}$
*A61K 8/99* $^{(2017.01)}$  *A61Q 11/00* $^{(2006.01)}$
*C12R 1/225* $^{(2006.01)}$

(52) Cooperative Patent Classification (CPC):
**A23L 33/135; A23C 9/1234; A61K 8/99;**
**A61Q 11/00; C12N 1/20; C12N 1/205;**
A23V 2400/175; C12R 2001/225

(86) International application number:
**PCT/CN2020/101662**

(87) International publication number:
**WO 2021/114659 (17.06.2021 Gazette 2021/24)**

(54) **LACTOBACILLUS RHAMNOSUS X253 BENEFICIAL TO ORAL HEALTH, SEPARATION AND PURIFICATION METHOD THEREFOR AND USE THEREOF**

LACTOBACILLUS RHAMNOSUS X253 FÜR DIE ORALE GESUNDHEIT, TRENNUNGS- UND REINIGUNGSVERFAHREN DAFÜR UND VERWENDUNG DAVON

LACTOBACILLUS RHAMNOSUS X253 BÉNÉFIQUE POUR LA SANTÉ BUCCALE, SON PROCÉDÉ DE SÉPARATION ET DE PURIFICATION, ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2019 CN 201911275130**
**03.04.2020 CN 202010258772**

(43) Date of publication of application:
**19.10.2022 Bulletin 2022/42**

(73) Proprietor: **Junlebao Dairy Group Co., Ltd.**
**Shijiazhuang, Hebei 050000 (CN)**

(72) Inventors:
• **FENG, Lili**
**Shijiazhuang, Hebei 050000 (CN)**
• **ZHANG, Dong**
**Shijiazhuang, Hebei 050000 (CN)**
• **WEI, Lihua**
**Shijiazhuang, Hebei 050000 (CN)**
• **WANG, Shijie**
**Shijiazhuang, Hebei 050000 (CN)**
• **XUE, Yuling**
**Shijiazhuang Hebei 050000 (CN)**
• **XUN, Yiping**
**Shijiazhuang, Hebei 050000 (CN)**
• **FENG, Xiaoying**
**Shijiazhuang, Hebei 050000 (CN)**
• **KANG, Zhiyuan**
**Shijiazhuang, Hebei 050000 (CN)**
• **ZHU, Hong**
**Shijiazhuang, Hebei 050000 (CN)**

(74) Representative: **reuteler & cie SA**
**Chemin de la Vuarpillière 29**
**1260 Nyon (CH)**

(56) References cited:
WO-A1-2015/159125    WO-A1-2017/050980
CN-A- 108 048 347    CN-A- 108 048 347
CN-A- 108 514 112    CN-A- 109 536 415
CN-A- 111 363 704    US-A1- 2004 101 495

**(Cont. next page)**

- **DOUILLARD FRAN�OIS P. ET AL: "Comparative Genomic and Functional Analysis of 100 Lactobacillus rhamnosus Strains and Their Comparison with Strain GG", vol. 9, no. 8, 16 January 2013 (2013-01-16), pages e1003683, XP055912801, Retrieved from the Internet <URL:https://www.ncbi.nlm.nih.gov/pmc/articles/PMC3744422/pdf/pgen.1003683.pdf> DOI: 10.1371/journal.pgen.1003683**
- **S. BISWAS, L. TURNER, I. BISWAS: "Lactobacillus rhamnosus LRB mediated inhibition of oral streptococci", MOLECULAR ORAL MICROBIOLOGY, vol. 33, no. 5, 7 August 2018 (2018-08-07), pages 396 - 405, XP055820176, ISSN: 2041-1006, DOI: 10.1111/omi.12242**

Remarks:
The complete document including Reference Table(s) and the Sequence Listing(s) can be downloaded from the EPO website

## Description

## Technical Field

The present invention belongs to the field of bioengineering, and relates to a strain of lactobacillus, in particular to a strain of Lactobacillus rhamnosus X253 beneficial to oral health, a separation and purification method and use thereof.

## Background Art

Oral cavity is the first gateway for human health. The oral environment of human is a complex ecosystem containing a variety of microorganisms, to which oral health is closely related. Under normal conditions, there is a dynamic balance among the microorganisms in the oral cavity and between the microorganisms and the host, and they jointly participate in the metabolism, immunity and nutrition of the body and jointly maintain oral health; however, if the dynamic balance of the oral microecology is broken under the influences of various factors, relevant oral diseases, such as dental caries, periodontal disease, halitosis, and oral candidiasis, etc., may occur.

Probiotic bacteria play an important role in the prevention and treatment of oral diseases. The most common probiotic bacteria in the oral microenvironment are Lactobacillus, which account for about 1% of the culturable microorganisms in the oral cavity. The most common lactobacillus include L. rhamnosus, L. Reuteri, L. salivarius, L. casei, L. acidophilus, L. fermentum and L. plantarllm. Therefore, it is of great significance and application value to screen a strain that can inhibit harmful bacteria in the oral cavity, has strong acid resistance, and maintains stable viable count during shelf life.

## Contents of the Invention

The technical problem to be solved by the present invention is to provide Lactobacillus rhamnosus X253 beneficial to oral health, which is separated and screened from fermented milk from Xinjiang, can inhibit harmful bacteria in the oral cavity, has strong acid resistance and has ability to maintain viable count, thereby can play a probiotic role and improve the oral environment.

Another object of the present invention is to provide a method for separation and purification of Lactobacillus rhamnosus X253 beneficial to oral health.

Another object of the present invention is to provide use of Lactobacillus rhamnosus X253 beneficial to oral health.

To attain the objects described above, the present invention employs the following technical solution: Lactobacillus rhamnosus X253 beneficial to oral health, which was isolated and screened from fermented milk from Xinjiang, and its strain has been preserved in China General Microbiological Culture Collection Center (address: No. 3, Courtyard No. 1, Beichen West Road, Chaoyang District, Beijing) on Aug. 20, 2019 with a preservation number CGMCC No. 18404.

As a limitation, the Lactobacillus rhamnosus X253 beneficial to oral health has the following 16SrRNA sequence:

CTTAGACGGCTCGCTCCCTAAAAGGGTTACGCCACCGGCTTCGGGTGTTACAAACTCTC

ATGGTGTGACGGGCGGTGTGTACAAGGCCCGGGAACGTATTCACCGCGGCGTGCTGAT

CCGCGATTACTAGCGATTCCGACTTCGTGTAGGCGAGTTGCAGCCTACAGTCCGAACTG

AGAATGGCTTTAAGAGATTAGCTTGACCTCGCGGTCTCGCAACCTCGTTGTACCCATCC

ATTGTAGCACGTGTGTAGCCCAGGTCATAAGGGGCATGATGATTTGACGTCATCCCCAC

CTTCCTCCGGTTTGTCACCGGCAGGTCTTACTAGAGTGCCCAACTAAATGCTGGCAACT

AGTCATAAGGGTTGCGCTCGTTGCGGGACTTAACCCAACATCTCACGACACGAGCTGA

CGACAACCATGCACCACCTGTCATTTTGCCCCCGAAGGGGAAACCTGATCTCTCAGGTG

ATCAAAGATGTCAAGACCTGGTAAGGTTCTTCGCGTTGCTTCGAATTAAACCACATGC

TCCACCGCTTGTGCGGGCCCCGTCAATTCCTTTGAGTTTCAACCTTGCGGTCGTACTCC

CAGGCGGAATGCTTAATGCGTTAGCTGCGGCACTGAAGGGCGGAAACCCTCCAACACC

TAGCATTCATCGTTTACGGCATTGGACTACCAGGGTATCTAATCCTGTTCGCTACCCAT

GCTTTCGAGCCTCAGCGTCAGTTACAGACCAGACAGCCGCCTTCGCCACTGGTGTTCTT

CCATATATCTACGCATTTCACCAGCTACACATGGAGTTCCACTGTCCTCTTCTGCACTCA

AGTTTCCCAGTTTCCGATGCACTTCCTCGGTTAAGCCGAGGGCTTTCACATCAGACTTA

AAAAACCGCCTGCGCTCGCTTTACGCCCAATAAATCCGGATAACGCTTGCCACCTACGT

ATTACCGCGGCTGCTGGCACGTAGTTAGCCGTGGCTTTCTGGTTGGATACCGTCACGCC

GACAACAGTTACTCTGCCGACCATTCTTCTCCACAACAGAGTTTTACGACCCGAAAGCC

TTCTTCACTCACGCGGCGTTGCTCCATCAGACTTGCGTCCATTGTGGAAGATTCCCTACT

GCTGCCTCCCGTAGGAGTTTGGGCCGTGTCTCAGTCCCAAATGTGGCCGATCAACCTCT

CAGTTCGGCTACGTATCATTGCCTTGGTGAGCCGTTACCTCACCAACTAGCTAATACGC

CGCGGGTCCATCCAAAAGCGATAGCTTACGCCATCTTTCAGCCAAGAACCATGCGGTTC

TTGGATTTATGCGGTATTAGCATCTGTTTCCAAATGTTATCCCCCACTTAAGGGCAGGTT

ACCCACGTGTTACTCACCCGTCGCCACTCGTTCAAAATTAAATCAAGATGCAAGCACCT

TTCAATAATCAGAACTCGTTCGACTTGCATGTAT.

As another limitation, the Lactobacillus rhamnosus X253 beneficial to oral health has the following Phes gene sequence:

GGGGGAAAAATAGCCGCACGCAAACATTCGGATGCACCATGCCGGCACCGAGAACTTC

AATCCAGCCGGTATACTTGCAAACAGGGCAACCCTTGCCACCGCAGCGGAAGCAGGAA

ACGTCCACTTCTACAGACGGTTCTGTAAACGGAAAATAGCTCGGGCGCAGGCGAATCG

TCCGATCAGCGCCAAACACATGCTGACACATGGCGAGTAACGTCCCCTTAAGATCAGC

CATTGTAATATGCTTGTCGATCACCAGACCTTCCATCTGATGGAACTGGTGGCTATGTG

TGGCATCATCATCATCACGCCGATAAACCACGCCCGGACTGATCATTTTCAGCGGGCCC

TTAGTAAAATCATGTTTCTCCATCGTCCGTGCCTGCATCGGGCTGGTTTGGGAACGCAT

CAGCAACTCATTGGTAATATAAAAGTTTCCTGCATATCACGAGCATGGATGACGCCGA.

The present invention further provides a method for separating and purifying the above Lactobacillus rhamnosus X253 beneficial to oral health, which comprises the following steps:

(I) sample collection
adding 25 mL fermented milk from Xinjiang into 200-250 mL normal saline, and mixing extensively to obtain a collected sample;

(II) sample enrichment
adding 2 mL collected sample to 80-120 mL MRS liquid culture medium, and culturing at 32-42 °C for 64-80 hours to obtain a culture solution;

(III) strain separation

Taking 1 mL culture solution and diluting for 10 times with 0.9% (w/v, wherein, w represents weight, v represents volume) sterile normal saline, and then carry out gradient dilution of $10^{-1}$, $10^{-2}$, $10^{-3}$, $10^{-4}$ and $10^{-5}$ times with normal saline respectively to obtain bacterial suspensions;

taking and dissolving a MRS solid culture medium and pouring the dissolved MRS culture medium into sterilized Petri dishes to prepare sterile plates; after cooling and full solidification, pipetting 0.1 mL bacterial suspensions at different concentrations and coating the bacterial suspensions on the sterile plates respectively;

performing anaerobic culture at 32-42 °C for 64-80 hours;

after typical colonies appear on the plates, selecting corresponding single colony for the next step of strain purification, according to the colony characteristics of standard lactic acid bacteria with reference to images in relevant literature;

(IV) strain purification
picking the selected single colony and streak-inoculating onto a sterile plate, and culturing in an aerobic environment at 32-42 °C for 64-80 hours; then, picking the single colony on the sterile plate and streak-inoculating onto another sterile plate, and culturing in an aerobic environment at 32-42 °C for 64-80 hours; in that way, culturing continuously for three times till the morphology of the colony in the culture medium is consistent; after observing the single cell morphology of the strain with a microscope, obtaining a pure cultured strain; then picking the colony with an inoculating loop and inoculating it in the MRS liquid culture medium and culturing at 37 °C for 24 hours.

As a limitation, the strain is preserved by taking 800 μL pure cultured strain and 50% sterile glycerol and loading them into a strain preservation tube, mixing them homogeneously and preserving the mixture at -70 °C, meanwhile inoculating on test-tube slant of the MRS solid culture medium for temporary preservation.

As a limitation, the MRS liquid culture medium mainly consists of the following raw materials: 8-12 g casein peptone, 8-12 g beef extract, 4-6 g yeast extract, 16-24 g glucose, 4-6 g sodium acetate, 1.5-2.5 g diamine citrate, 0.8-1.2 g Tween-80, 1.5-2.5 g $K_2HPO_4$, 0.1-0.3 g $MgSO_4 \cdot 7H_2O$, 0.04-0.06 g $MnSO_4 \cdot 7H_2O$, and 1,000 mL distilled water.

As a limitation, the MRS solid culture medium mainly consists of the following raw materials: 8-12 g casein peptone, 8-12 g beef extract, 4-6 g yeast extract, 16-24 g glucose, 4-6 g sodium acetate, 1.5-2.5 g diamine citrate, 0.8-1.2 g Tween-80, 1.5-2.5 g $K_2HPO_4$, 0.1-0.3 g $MgSO_4 \cdot 7H_2O$, 0.04-0.06 g $MnSO_4 \cdot 7H_2O$, 13-17 g agar, and 1,000 mL distilled water.

The present invention further provides use of the Lactobacillus rhamnosus X253 beneficial to oral health, which can inhibit harmful bacteria in the oral cavity and can be used to prepare a product for improving oral health.

As a limitation, the product for improving oral health is toothpaste, mouth wash, probiotic buccal tablets, lactobacillus beverage or probiotic fermented milk.

With the technical solution described above, compared with the prior art, the present invention achieves the following technical progresses:

The strain adopted by the present invention has strong acid resistance and high tolerance to gastric fluid and intestinal fluid, and can reach the intestinal tract, thereby plays a probiotic role; the strain adopted by the present invention is capable of inhibiting harmful strains in the oral cavity, can effectively improve the oral environment and prevent and treat oral diseases; the strain adopted by the present invention has a strong ability to maintain the viable count in the shelf life, and can keep products stable in the shelf life.

**Description of Drawings**

Fig. 1 is a schematic diagram of the result of an experiment for the characteristics of in vitro adhesion to intestinal epithelial cells in example 2 of the present invention;

Fig. 2 is a schematic diagram of fermentation curve of an experiment for fermentation characteristics in example 2 of the present invention.

**Embodiments**

Hereunder preferred examples of the present invention will be detailed with reference to the accompanying drawings. It should be understood that the preferred examples described here are only provided to describe and explain the present invention, and shall not be used to limit the present invention.

Example 1: Lactobacillus rhamnosus X253 beneficial to oral health, and separation and purification method thereof

I. Strain

The Lactobacillus rhamnosus X253 beneficial to oral health was separated and screened from fermented milk from Xinjiang, and its strain has been preserved in China General Microbiological Culture Collection Center, with a preservation number CGMCC NO. 18404.

II. Method for separating and purifying the Lactobacillus rhamnosus X253 beneficial to oral health

The following steps are executed sequentially:

(I) Sample collection
25 mL fermented milk from Xinjiang was added into 200-250 mL normal saline, and mixed extensively to obtain a collected sample;

(II) Sample enrichment

2 mL collected sample was taken and added to 80-120 mL MRS liquid culture medium, and cultured at 32-42 °C for 64-80 hours to obtain a culture solution;

The MRS liquid culture medium mainly consists of the following raw materials: 8-12 g casein peptone, 8-12 g beef extract, 4-6 g yeast extract, 16-24 g glucose, 4-6 g sodium acetate, 1.5-2.5 g diamine citrate, 0.8-1.2 g Tween-80, 1.5-2.5 g $K_2HPO_4$, 0.1-0.3 g $MgSO_4 \cdot 7H_2O$, 0.04-0.06 g $MnSO_4 \cdot 7H_2O$, and 1,000 mL distilled water.

(III) Strain separation

1 mL culture solution was taken and diluted for 10 times with 0.9% (w/v) sterile normal saline, and then gradient dilution of $10^{-1}$, $10^{-2}$, $10^{-3}$, $10^{-4}$ and $10^{-5}$ times was carried out with the normal saline respectively to obtain bacterial suspensions;

A MRS solid culture medium was taken and dissolved, then poured into sterilized Petri dishes to prepare sterile plates; after cooling and full solidification, 0.1 mL bacterial suspensions at different concentrations were pipetted and coated on the sterile plates respectively;

An anaerobic culture was performed at 32-42 °C for 64-80 hours;

After typical colonies appeared on the plates, corresponding single colony is selected for the next step of strain purification, according to the colony characteristics of standard lactic acid bacteria with reference to images in relevant literature;

(IV) Strain purification
The selected single colony was picked and streak-inoculated onto a sterile plate, and cultured in an aerobic environment at 32-42 °C for 64-80 hours; then, a single colony on the sterile plate was picked and streak-inoculated onto another sterile plate, and cultured in an aerobic environment at 32-42 °C for 64-80 hours; in that way, the culture was carried out continuously for three times till the morphology of the colony in the culture medium was consistent;

after the single cell morphology of the strain was observed with a microscope, a pure cultured strain was obtained; then the colony was picked with an inoculating loop and inoculated in the MRS liquid culture medium and cultured at 37 °C for 24 hours, thus a proliferated pure cultured strain was obtained. The MRS solid culture medium mainly consists of the following raw materials: 8-12 g casein peptone, 8-12 g beef extract, 4-6 g yeast extract, 16-24 g glucose, 4-6 g sodium acetate, 1.5-2.5 g diamine citrate, 0.8-1.2 g Tween-80, 1.5-2.5 g $K_2HPO_4$, 0.1-0.3 g $MgSO_4 \cdot 7H_2O$, 0.04-0.06 g $MnSO_4 \cdot 7H_2O$, 13-17 g agar, and 1,000 mL distilled water.

(V) Preservation of strain

800 μL pure cultured strain and 50% sterile glycerol were taken and loaded into a strain preservation tube, mixed homogeneously and preserved at -70°C, at the same time inoculation on test-tube slant of MRS solid culture medium was performed for temporary preservation.

The MRS solid culture medium mainly consists of the following raw materials: 8-12 g casein peptone, 8-12 g beef extract, 4-6 g yeast extract, 16-24 g glucose, 4-6 g sodium acetate, 1.5-2.5 g diamine citrate, 0.8-1.2 g Tween-80, 1.5-2.5 g $K_2HPO_4$, 0.1-0.3 g $MgSO_4 \cdot 7H_2O$, 0.04-0.06 g $MnSO_4 \cdot 7H_2O$, 13-17 g agar, and 1,000 mL distilled water.

Example 2: Bacteriological characteristics of the Lactobacillus rhamnosus X253 beneficial to oral health

I. Basic characteristics

The basic characteristics of X253 are shown in Table 1.

Table 1. Basic Characteristics of X253

| Test item | Result | Test item | Result | Test item | Result |
|---|---|---|---|---|---|
| Gram staining | Positive | Cell morphology | Rod shape | Catalase | - |
| Oxidase | - | Sporulation | - | | |

It can be seen from Table 1 that X253 is a Gram-positive, rod-shaped, spore-free, catalase-negative and oxidase-negative strain.

II. Experiment for sugar fermentation characteristics

A single colony of the separated and purified strain was picked, streaked on a plate, and cultured at 37 °C for 48 hours, and the strain was picked with an inoculating loop and inoculated into a sugar fermentation tube, and cultured at 37 °C for 48 hours, and the color change was observed. The result is shown in Table 2.

Table 2. Identification Result of X253

| Glycerol | - | Inositol | - | Inulin | - |
|---|---|---|---|---|---|
| Erythritol | - | Mannitol | + | Melizitose | + |
| D-arabinose | - | Sorbitol | + | Raffinose | - |
| L-arabinose | - | α-methyl-D-mannoside | - | Starch | - |
| D-ribose | + | α-methyl-D-glucoside | + | Glycogen | - |
| D-xylose | - | N-acetyl-glucosamine | + | Xylitol | - |
| L-xylose | - | Amygdalin | + | Gentiobiose | |
| Adonitol | - | Arbutin | + | D-turanose | + |
| β-methyl-D-xyloside | - | Esculin | + | D-lyxose | - |
| D-galactose | + | Salicin | + | D-tagatose | + |
| D-glucose | + | Cellobiose | + | D-fucose | - |
| D-fructose | + | Maltose | + | L-fucose | - |
| D-mannose | + | Lactose | + | D-arabitol | - |

(continued)

| L-sorbose | + | Melibiose | - | L-arabitol | - |
|---|---|---|---|---|---|
| L-rhamnose | + | Sucrose | + | Gluconate | - |
| Dulcitol | - | Trehalose | + | 2-keto-gluconate | - |
| Note: "+" means use through fermentation; "-" means no use through fermentation. | | | | | |

III. Molecular biological identification

The strain was subjected to molecular biological identification, and the 16SrRNA sequence of X253 was as follows:

CTTAGACGGCTCGCTCCCTAAAAGGGTTACGCCACCGGCTTCGGGTGTTACAAACTCTC

ATGGTGTGACGGGCGGTGTGTACAAGGCCCGGGAACGTATTCACCGCGGCGTGCTGAT

CCGCGATTACTAGCGATTCCGACTTCGTGTAGGCGAGTTGCAGCCTACAGTCCGAACTG

AGAATGGCTTTAAGAGATTAGCTTGACCTCGCGGTCTCGCAACCTCGTTGTACCCATCC

ATTGTAGCACGTGTGTAGCCCAGGTCATAAGGGGCATGATGATTTGACGTCATCCCCAC

CTTCCTCCGGTTTGTCACCGGCAGGTCTTACTAGAGTGCCCAACTAAATGCTGGCAACT

AGTCATAAGGGTTGCGCTCGTTGCGGGACTTAACCCAACATCTCACGACACGAGCTGA

CGACAACCATGCACCACCTGTCATTTTGCCCCCGAAGGGGAAACCTGATCTCTCAGGTG

ATCAAAGATGTCAAGACCTGGTAAGGTTCTTCGCGTTGCTTCGAATTAAACCACATGC

TCCACCGCTTGTGCGGGCCCCGTCAATTCCTTTGAGTTTCAACCTTGCGGTCGTACTCC

CAGGCGGAATGCTTAATGCGTTAGCTGCGGCACTGAAGGGCGGAAACCCTCCAACACC

TAGCATTCATCGTTTACGGCATTGGACTACCAGGGTATCTAATCCTGTTCGCTACCCAT

GCTTTCGAGCCTCAGCGTCAGTTACAGACCAGACAGCCGCCTTCGCCACTGGTGTTCTT

CCATATATCTACGCATTTCACCAGCTACACATGGAGTTCCACTGTCCTCTTCTGCACTCA

AGTTTCCCAGTTTCCGATGCACTTCCTCGGTTAAGCCGAGGGCTTTCACATCAGACTTA

AAAAACCGCCTGCGCTCGCTTTACGCCCAATAAATCCGGATAACGCTTGCCACCTACG

T

ATTACCGCGGCTGCTGGCACGTAGTTAGCCGTGGCTTTCTGGTTGGATACCGTCACGC
C

GACAACAGTTACTCTGCCGACCATTCTTCTCCACAACAGAGTTTTACGACCCGAAAG
CC

TTCTTCACTCACGCGGCGTTGCTCCATCAGACTTGCGTCCATTGTGGAAGATTCCCTA
CT

GCTGCCTCCCGTAGGAGTTTGGGCCGTGTCTCAGTCCCAAATGTGGCCGATCAACCT
CT

CAGTTCGGCTACGTATCATTGCCTTGGTGAGCCGTTACCTCACCAACTAGCTAATACG
C

CGCGGGTCCATCCAAAAGCGATAGCTTACGCCATCTTTCAGCCAAGAACCATGCGGTT
C

TTGGATTTATGCGGTATTAGCATCTGTTTCCAAATGTTATCCCCCACTTAAGGGCAGGT
T

ACCCACGTGTTACTCACCCGTCGCCACTCGTTCAAAATTAAATCAAGATGCAAGCACC
T

TTCAATAATCAGAACTCGTTCGACTTGCATGTAT

The Phes gene sequence of X253 was as follows:

GGGGGAAAAATAGCCGCACGCAAACATTCGGATGCACCATGCCGGCACCGAGAACTTC

AATCCAGCCGGTATACTTGCAAACAGGGCAACCCTTGCCACCGCAGCGGAAGCAGGAA

ACGTCCACTTCTACAGACGGTTCTGTAAACGGAAAATAGCTCGGGCGCAGGCGAATCG

TCCGATCAGCGCCAAACACATGCTGACACATGGCGAGTAACGTCCCCTTAAGATCAGC

CATTGTAATATGCTTGTCGATCACCAGACCTTCCATCTGATGGAACTGGTGGCTATGTG

TGGCATCATCATCATCACGCCGATAAACCACGCCCGGACTGATCATTTTCAGCGGGCCC

TTAGTAAAATCATGTTTCTCCATCGTCCGTGCCTGCATCGGGCTGGTTTGGGAACGCAT

CAGCAACTCATTGGTAATATAAAAGTTTCCTGCATATCACGAGCATGGATGACGCCGA

III. Experiment on the characteristics of in vitro adhesion to intestinal epithelial cells

(I) Experimental method:

1. 1 mL activated Lactobacillus rhamnosus X253 strain was taken and centrifuged at 4,000 rpm for 5 minutes. According to the empirical value, the concentration of the bacterial suspension is 109 CFU/mL, the bacteria was washed twice with PBS, and then resuspended with 1 mL DMEM in a 5 mL centrifuge tube.

2. The original cell culture solution in a 24-well plate was drawn out, and washed with PBS for one time.

3. Adding bacteria: each well contains 1 mL bacterial suspension, and two duplicates were prepared for each concentration of bacterial suspension.

4. Incubation: the sample is placed in a carbon dioxide incubator at 37 °C and incubates for 3 hours.

5. The total number of colonies in the initial bacterial suspension in the adhesion experiment is counted.

6. Counting: after the incubation, the bacteria were washed with PBS for 4 times and lysed with 1 mL sterilized 1% (v/v) Triton x 100. After the lysis, the solution was poured on the plates according to the dilution gradient in the following table (dilution method: adding 300$\mu$L into 2.7 mL normal saline). The sample was incubated at 37 °C for 3 days, and the result is recorded.

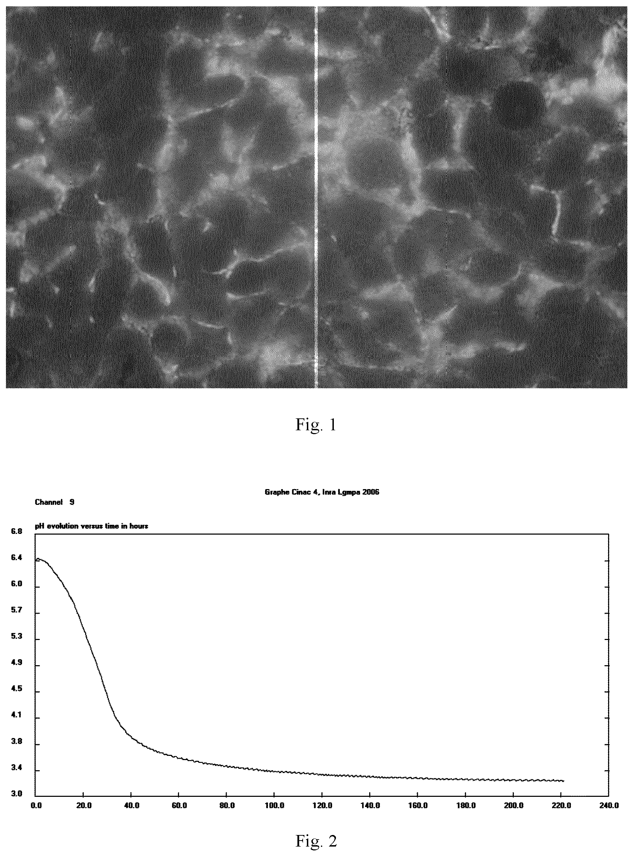

(II) The result of the experiment on the characteristics of in vitro adhesion to intestinal epithelial cells is shown in Fig. 1.
IV. Fermentation characteristics of the strain

(I) Experimental method

Activated Lactobacillus rhamnosus X253 strain was inoculated in 1% inoculation amount into a MRS liquid culture medium, and cultured at 37 °C for 48 hours; the cultured strain was centrifuged at 3,500 rpm (at 4 °C) for 10 minutes, washed once with normal saline, and suspended in sterilized skim milk (sterilization conditions: 115 °C, 15 minutes) as a seed solution for later use. The above seed solution was inoculated in 1% inoculation amount into sterilized skim milk (sterilization conditions: 115 °C, 15 minutes), and the change of the pH of the Lactobacillus rhamnosus X253 in the sterilized milk was monitored online.

(II) The strain fermentation curve is shown in Fig. 2.

Example 3. Gastric acid and intestinal fluid tolerance test

I. Experimental method

(I) Tested strain: X253

(II) Preparation of artificial digestive fluid

Artificial gastric fluid: 0.2 g/100 mL NaCl and 0.35 g/100 mL pepsin, after the pH was adjusted to 3.0 with 1 mol/L HCl, the artificial gastric fluid was filtered and sterilized for later use.

Artificial intestinal fluid: the following fluid a and fluid b were mixed at a ratio of 2:1 to prepare artificial intestinal fluid.

a. Pancreatic fluid: 1.1 g/100 mL sodium bicarbonate, 0.2g/100ml NaCl, and 0.1 g/100 mL trypsin, after the pH was adjusted to 8.0, the pancreatic fluid was filtered and sterilized for later use.

b. Bile fluid: 0.9 g/100 mL Bile Salts (Difco), after the pH was adjusted to 8.0, the bile fluid was filtered and sterilized for later use.

(III) After the strain to be tested was activated for 3 generations, 1mL strain was taken and placed in 9 mL filtered and sterilized artificial gastric fluid with pH=3.0, the solution was shaken homogeneously and cultured at 37 °C; samples were taken at the beginning of culturing and 2 hours culturing respectively, and the viable counts were determined Then, 1 mL culture solution which had been digested in the artificial gastric fluid with pH=3.0 for 2 hours was taken respectively, inoculated into 9 mL filtered and sterilized artificial intestinal fluid with pH=8.0 respectively, and further cultured at 37 °C; the viable count was measured at 0 hour and 4 hours respectively.

$$\text{Viability } (\%) = (\text{cfu N1/cfu N0}) \times 100\%$$

where, N1 - the viable count after treatment with the artificial digestive fluid for 6 hours; N0 - the viable count after treatment with the artificial digestive fluid for 0 hour.

(IV) The experimental result is shown in the following Table 3:

Table 3. Gastric Fluid and Intestinal Fluid Tolerance Test Result of X253

| Strain | 0h | 2h | 6h | Viability in gastric fluid | Viability in intestinal fluid | Viability in digestive fluid |
|--------|----|----|----|----------------------------|-------------------------------|------------------------------|
| X253 | $8.6 \times 10^8$ | $6.88 \times 10^7$ | $8.0 \times 10^3$ | 80% | 0.12% | 0.09% |

Example 4. Experiment on inhibition of harmful bacteria in the oral cavity

Using Streptococcus mutans, Streptococcus gordonii, Porphyromonas gingivalis, Fusobacterium nucleatum and Actinobacillus actinomycetem comitans as indicator bacteria, the sizes of the inhibition zones of X253 against the five pathogenic strains were detected, so as to judge the bacteriostatic ability.

I. Bacteriostatic experiment of X253 against Streptococcus mutans and Streptococcus gordonii

(I) Seed activation and culture

Streptococcus mutans and Streptococcus gordonii were inoculated in 5% inoculum amount into BHI liquid culture medium respectively, and then cultured in an anaerobic environment at 37 °C for 20 hours. Usually a bacteria solution activated continuously for three generations was used for the bacteriostatic test.

(II) Preparation of bacterial powder solution

2 g bacterial powder of X253 was mixed with 8 g sterile purified water to prepare a 20% bacterial powder solution.

(III) An Oxford cup double-layer plate method was employed.

(1) 2.0% pure agar culture medium was cooled to about 50 °C, 10 mL pure agar culture medium was taken and poured into a sterile plate, mixed homogeneously and flattened, and dried in a clean workbench;

(2) A sterilized Oxford cup was picked up with sterile tweezers and placed in a plate with pure agar, and set for 10 minutes;

(3) 1 mL Streptococcus mutans or 2 mL Streptococcus gordonii was added into 100 mL BHI semi-solid culture medium which has been cooled to 50 °C and mixed extensively;

(4) 10 mL BHI semi-solid culture medium was poured on the plate where the Oxford cup was placed; after solidification, the Oxford cup was clamped out to leave an Oxford cup hole;

(5) 100 μL 20% oral probiotic bacteria powder solution was injected into the hole, and incubated at 37 °C for 18-20 hours; then the diameter of the inhibition zone was measured with a vernier caliper. Five groups of parallel experiments were set up.

(6) Erythromycin susceptibility paper was used as a positive control and sterile purified water was used as a negative control.

II. Bacteriostatic experiment of X253 against Porphyromonas gingivalis, Fusobacterium nucleatum and Actinobacillus actinomycetem comitans

(I) Seed activation and culture

Porphyromonas gingivalis, Fusobacterium nucleatum and Actinobacillus actinomycetem comitans were inoculated in 5% inoculum amount into a modified TSB liquid culture medium respectively, and cultured in an anaerobic environment at 37 °C for 5 days; usually a bacteria solution activated continuously for three generations was used for the bacteriostatic test.

(II) Preparation of bacterial powder solution

2 g probiotic bacteria powder of X253 was mixed with 8 g sterile purified water to prepare a 20% bacterial powder solution.

(III) An Oxford cup method was employed.

(1) The modified TSA culture medium was cooled to about 50 °C, then 20 mL modified TSA culture medium was taken and poured into a sterile plate and dried in a clean workbench;
(2) 0.1 mL Fusobacterium nucleatum, Porphyromonas gingivalis and Actinobacillus actinomycetem comitans were added to the plate with the modified TSA, and then quickly and evenly coated with a sterile spreader;
(3) A sterilized Oxford cup was picked with sterile tweezers and placed in a plate coated with different pathogenic bacteria;

14

(4) 100 μL 20% oral probiotic bacteria powder solution was injected into the Oxford cup, and cultured in an anaerobic environment at 37 °C for 7 days; then the diameter of the inhibition zone was measured with a vernier caliper. Three groups of parallel experiments were set up.

(5) Erythromycin susceptibility paper was used as a positive control and sterile purified water was used as a negative control.

III. The experimental results are shown in Table 4.

Table 4. Bacteriostatic Ability of Probiotic Bacteria Powder of X253 against Harmful Oral Bacteria

|  | Streptococcus mutans | Streptococcus gordonii | Porphyromonas gingivalis | Actinobacillus actinomycetem comitans | Fusobacterium nucleatum |
|---|---|---|---|---|---|
| Negative control | 0±0.0 | 0±0.0 | 0±0.0 | 0±0.0 | 0±0.0 |
| X253 | 15.36±0.3 | 17.23±1.1 | 15.26±1.0 | 14.82±1.1 | 15.32±0.56 |
| Erythromycin | 38.0±0.0 | 34.3±0.0 | >50 | 27.0±0.0 | 22.7±0.0 |

It can be seen from the above table that X253 can effectively inhibit harmful bacteria in the oral cavity. Therefore, it can be used to prepare toothpaste, mouthwash, probiotic buccal tablets, lactobacillus beverage, probiotic fermented milk and other products that can improve oral health. In the end, it should be noted that above mentioned are only preferred examples of the present invention, which are not used to limit the present invention. While the present invention has been described with reference to the above mentioned examples, those skilled in the art still can make modifications to the technical solution described in the above mentioned examples or make equivalent replacements to some technical features therein.

## Claims

1. Lactobacillus rhamnosus X253 beneficial to oral health, which was isolated and screened from fermented milk from Xinjiang, and preserved in China General Microbiological Culture Collection Center, the address of which is No. 3, Courtyard No. 1, Beichen West Road, Chaoyang District, Beijing, on Aug. 20, 2019 with a preservation number CGMCC No.18404.

2. The Lactobacillus rhamnosus X253 beneficial to oral health according to claim 1, **characterized in that**, its 16SrRNA sequence is as follows:

CTTAGACGGCTCGCTCCCTAAAAGGGTTACGCCACCGGCTTCGGGTGTTAC

AAACTCTCATGGTGTGACGGGCGGTGTGTACAAGGCCCGGGAACGTATTC

ACCGCGGCGTGCTGATCCGCGATTACTAGCGATTCCGACTTCGTGTAGGCG

AGTTGCAGCCTACAGTCCGAACTGAGAATGGCTTTAAGAGATTAGCTTGAC

CTCGCGGTCTCGCAACCTCGTTGTACCCATCCATTGTAGCACGTGTGTAGCC

CAGGTCATAAGGGGCATGATGATTTGACGTCATCCCCACCTTCCTCCGGTTT

GTCACCGGCAGGTCTTACTAGAGTGCCCAACTAAATGCTGGCAACTAGTCA

TAAGGGTTGCGCTCGTTGCGGGACTTAACCCAACATCTCACGACACGAGCT

GACGACAACCATGCACCACCTGTCATTTTGCCCCCGAAGGGGAAACCTGAT

CTCTCAGGTGATCAAAAGATGTCAAGACCTGGTAAGGTTCTTCGCGTTGCT

TCGAATTAAACCACATGCTCCACCGCTTGTGCGGGCCCCCGTCAATTCCTTT

GAGTTTCAACCTTGCGGTCGTACTCCCAGGCGGAATGCTTAATGCGTTAGC

TGCGGCACTGAAGGGCGGAAACCCTCCAACACCTAGCATTCATCGTTACG

GCATTGGACTACCAGGGTATCTAATCCTGTTCGCTACCCATGCTTTCGAGCC

TCAGCGTCAGTTACAGACCAGACAGCCGCCTTCGCCACTGGTGTTCTTCCA

TATATCTACGCATTTCACCAGCTACACATGGAGTTCCACTGTCCTCTTCTGC

ACTCAAGTTTCCCAGTTTCCGATGCACTTCCTCGGTTAAGCCGAGGGCTTT

CACATCAGACTTAAAAAACCGCCTGCGCTCGCTTTACGCCCAATAAATCCG

GATAACGCTTGCCACCTACGTATTACCGCGGCTGCTGGCACGTAGTTAGCC

GTGGCTTTCTGGTTGGATACCGTCACGCCGACAACAGTTACTCTGCCGACC

ATTCTTCTCCACAACAGAGTTTTACGACCCGAAAGCCTTCTTCACTCACGC

GGCGTTGCTCCATCAGACTTGCGTCCATTGTGGAAGATTCCCTACTGCTGCC

TCCCGTAGGAGTTTGGGCCGTGTCTCAGTCCCAAATGTGGCCGATCAACCT

CTCAGTTCGGCTACGTATCATTGCCTTGGTGAGCCGTTACCTCACCAACTAG

CTAATACGCCGCGGGTCCATCCAAAAGCGATAGCTTACGCCATCTTTCAGCC

AAGAACCATGCGGTTCTTGGATTTATGCGGTATTAGCATCTGTTTCCAAATG

TTATCCCCCACTTAAGGGCAGGTTACCCACGTGTTACTCACCCGTCGCCACT

CGTTCAAAATTAAATCAAGATGCAAGCACCTTTCAATAATCAGAACTCGTT

CGACTTGCATGTAT.

3. The Lactobacillus rhamnosus X253 beneficial to oral health according to claim 1, **characterized in that**, its Phes genetic sequence is as follows:

GGGGGAAAAATAGCCGCACGCAAACATTCGGATGCACCATGCCGGCACCG

AGAACTTCAATCCAGCCGGTATACTTGCAAACAGGGCAACCCTTGCCACCG

CAGCGGAAGCAGGAAACGTCCACTTCTACAGACGGTTCTGTAAACGGAAA

ATAGCTCGGGCGCAGGCGAATCGTCCGATCAGCGCCAAACACATGCTGAC

ACATGGCGAGTAACGTCCCCTTAAGATCAGCCATTGTAATATGCTTGTCGAT

CACCAGACCTTCCATCTGATGGAACTGGTGGCTATGTGTGGCATCATCATCA

TCACGCCGATAAACCACGCCCGGACTGATCATTTTCAGCGGGCCCTTAGTA

AAATCATGTTTCTCCATCGTCCGTGCCTGCATCGGGCTGGTTTGGGAACGCA

TCAGCAACTCATTGGTAATATAAAAGTTTCCTGCATATCACGAGCATGGAT

GACGCCGA.

4. Lactobacillus rhamnosus X253 according to any of claims 1-3 for use in the prevention and treatment of oral diseases and improving oral health.

5. Lactobacillus rhamnosus X253 for use according to claim 4, wherein said Lactobacillus rhamnosus X253 is included in a product for improving oral health, said product being selected from toothpaste, mouth wash, probiotic buccal tablets, lactobacillus beverage and probiotic fermented milk.

**Patentansprüche**

1. Lactobacillus rhamnosus X253, der sich günstig auf die Mundgesundheit auswirkt, aus fermentierter Milch aus Xinjiang isoliert und gescreent und am 20. August 2019 beim China General Microbiological Culture Collection Center, dessen Adresse No. 3, Courtyard No. 1, Beichen West Road, Chaoyang District, Beijing lautet, unter einer Hinterlegungsnummer CGMCC Nr. 18404 hinterlegt wurde.

2. Lactobacillus rhamnosus X253, der sich günstig auf die Mundgesundheit auswirkt, nach Anspruch 1, **dadurch gekennzeichnet, dass** seine 16SrRNA-Sequenz wie folgt lautet:

CTTAGACGGCTCGCTCCCTAAAAGGGTTACGCCACCGGCTTCGGGTGTTAC

AAACTCTCATGGTGTGACGGGCGGTGTGTACAAGGCCCGGGAACGTATTC

ACCGCGGCGTGCTGATCCGCGATTACTAGCGATTCCGACTTCGTGTAGGCG

AGTTGCAGCCTACAGTCCGAACTGAGAATGGCTTTAAGAGATTAGCTTGAC

CTCGCGGTCTCGCAACCTCGTTGTACCCATCCATTGTAGCACGTGTGTAGCC

CAGGTCATAAGGGGCATGATGATTTGACGTCATCCCCACCTTCCTCCGGTTT

GTCACCGGCAGGTCTTACTAGAGTGCCCAACTAAATGCTGGCAACTAGTCA

TAAGGGTTGCGCTCGTTGCGGGACTTAACCCAACATCTCACGACACGAGCT

GACGACAACCATGCACCACCTGTCATTTTGCCCCCGAAGGGGAAACCTGAT

CTCTCAGGTGATCAAAAGATGTCAAGACCTGGTAAGGTTCTTCGCGTTGCT

TCGAATTAAACCACATGCTCCACCGCTTGTGCGGGCCCCCGTCAATTCCTTT

GAGTTTCAACCTTGCGGTCGTACTCCCAGGCGGAATGCTTAATGCGTTAGC

TGCGGCACTGAAGGGCGGAAACCCTCCAACACCTAGCATTCATCGTTTACG

GCATTGGACTACCAGGGTATCTAATCCTGTTCGCTACCCATGCTTTCGAGCC

TCAGCGTCAGTTACAGACCAGACAGCCGCCTTCGCCACTGGTGTTCTTCCA

TATATCTACGCATTTCACCAGCTACACATGGAGTTCCACTGTCCTCTTCTGC

ACTCAAGTTTCCCAGTTTCCGATGCACTTCCTCGGTTAAGCCGAGGGCTTT

CACATCAGACTTAAAAAACCGCCTGCGCTCGCTTTACGCCCAATAAATCCG

GATAACGCTTGCCACCTACGTATTACCGCGGCTGCTGGCACGTAGTTAGCC

GTGGCTTTCTGGTTGGATACCGTCACGCCGACAACAGTTACTCTGCCGACC

ATTCTTCTCCACAACAGAGTTTTACGACCCGAAAGCCTTCTTCACTCACGC

GGCGTTGCTCCATCAGACTTGCGTCCATTGTGGAAGATTCCCTACTGCTGCC

TCCCGTAGGAGTTTGGGCCGTGTCTCAGTCCCAAATGTGGCCGATCAACCT

CTCAGTTCGGCTACGTATCATTGCCTTGGTGAGCCGTTACCTCACCAACTAG

CTAATACCGCGGGTCCATCCAAAAGCGATAGCTTACGCCATCTTTCAGCC

AAGAACCATGCGGTTCTTGGATTTATGCGGTATTAGCATCTGTTTCCAAATG

TTATCCCCCACTTAAGGGCAGGTTACCCACGTGTTACTCACCCGTCGCCACT

CGTTCAAAATTAAATCAAGATGCAAGCACCTTTCAATAATCAGAACTCGTT

CGACTTGCATGTAT.

3. Lactobacillus rhamnosus X253, der sich günstig auf die Mundgesundheit auswirkt, nach Anspruch 1, **dadurch gekennzeichnet, dass** seine genetische Phes-Sequenz wie folgt lautet:

GGGGGAAAAATAGCCGCACGCAAACATTCGGATGCACCATGCCGGCACCG

AGAACTTCAATCCAGCCGGTATACTTGCAAACAGGGCAACCCTTGCCACCG

CAGCGGAAGCAGGAAACGTCCACTTCTACAGACGGTTCTGTAAACGGAAA

ATAGCTCGGGCGCAGGCGAATCGTCCGATCAGCGCCAAACACATGCTGAC

ACATGGCGAGTAACGTCCCCTTAAGATCAGCCATTGTAATATGCTTGTCGAT

CACCAGACCTTCCATCTGATGGAACTGGTGGCTATGTGTGGCATCATCATCA

TCACGCCGATAAACCACGCCCGGACTGATCATTTTCAGCGGGCCCTTAGTA

AAATCATGTTTCTCCATCGTCCGTGCCTGCATCGGGCTGGTTTGGGAACGCA

TCAGCAACTCATTGGTAATATAAAAAGTTTCCTGCATATCACGAGCATGGAT

GACGCCGA.

4. Lactobacillus rhamnosus X253 nach einem der Ansprüche 1 bis 3 zur Verwendung bei der Prävention und Behandlung von Mundkrankheiten und zur Verbesserung der Mundgesundheit.

5. Lactobacillus rhamnosus X253 zur Verwendung nach Anspruch 4, wobei der Lactobacillus rhamnosus X253 in einem Produkt zur Verbesserung der Mundgesundheit enthalten ist, wobei das Produkt ausgewählt ist aus einer Zahnpasta, einer Mundspülung, probiotischen Mundtabletten, einem Lactobacillus-Getränk und probiotischer fermentierter Milch.

**Revendications**

1. Lactobacillus rhamnosus X253 bénéfique pour la santé buccale, qui a été isolé et criblé à partir de lait fermenté du Xinjiang, et conservé au Centre de collecte de la culture microbiologique générale de Chine, dont l'adresse est n°3, Parvis n°1, Route occidentale de Beichen, District de Chaoyang, Pékin, le 20 août 2019 avec le numéro d'enregistrement CGMCC n°18404.

2. Lactobacillus rhamnosus X253 bénéfique pour la santé buccale selon la revendication 1, **caractérisé en ce que** la séquence 16SrRNA est la suivante :

CTTAGACGGCTCGCTCCCTAAAAGGGTTACGCCACCGGCTTCGGGTGTTAC

AAACTCTCATGGTGTGACGGGCGGTGTGTACAAGGCCCGGGAACGTATTC

ACCGCGGCGTGCTGATCCGCGATTACTAGCGATTCCGACTTCGTGTAGGCG

AGTTGCAGCCTACAGTCCGAACTGAGAATGGCTTTAAGAGATTAGCTTGAC

CTCGCGGTCTCGCAACCTCGTTGTACCCATCCATTGTAGCACGTGTGTAGCC

CAGGTCATAAGGGGCATGATGATTTGACGTCATCCCCACCTTCCTCCGGTTT

GTCACCGGCAGGTCTTACTAGAGTGCCCAACTAAATGCTGGCAACTAGTCA

TAAGGGTTGCGCTCGTTGCGGGACTTAACCCAACATCTCACGACACGAGCT

GACGACAACCATGCACCACCTGTCATTTTGCCCCCGAAGGGGAAACCTGAT

CTCTCAGGTGATCAAAAGATGTCAAGACCTGGTAAGGTTCTTCGCGTTGCT

TCGAATTAAACCACATGCTCCACCGCTTGTGCGGGCCCCCGTCAATTCCTTT

GAGTTTCAACCTTGCGGTCGTACTCCCAGGCGGAATGCTTAATGCGTTAGC

TGCGGCACTGAAGGGCGGAAACCCTCCAACACCTAGCATTCATCGTTACG

GCATTGGACTACCAGGGTATCTAATCCTGTTCGCTACCCATGCTTTCGAGCC

TCAGCGTCAGTTACAGACCAGACAGCCGCCTTCGCCACTGGTGTTCTTCCA

TATATCTACGCATTTCACCAGCTACACATGGAGTTCCACTGTCCTCTTCTGC

ACTCAAGTTTCCCAGTTTCCGATGCACTTCCTCGGTTAAGCCGAGGGCTTT

CACATCAGACTTAAAAAACCGCCTGCGCTCGCTTACGCCCAATAAATCCG

GATAACGCTTGCCACCTACGTATTACCGCGGCTGCTGGCACGTAGTTAGCC

GTGGCTTTCTGGTTGGATACCGTCACGCCGACAACAGTTACTCTGCCGACC

ATTCTTCTCCACAACAGAGTTTTACGACCCGAAAGCCTTCTTCACTCACGC

GGCGTTGCTCCATCAGACTTGCGTCCATTGTGGAAGATTCCCTACTGCTGCC

TCCCGTAGGAGTTTGGGCCGTGTCTCAGTCCCAAATGTGGCCGATCAACCT

CTCAGTTCGGCTACGTATCATTGCCTTGGTGAGCCGTTACCTCACCAACTAG

CTAATACGCCGCGGGTCCATCCAAAAGCGATAGCTTACGCCATCTTTCAGCC

AAGAACCATGCGGTTCTTGGATTTATGCGGTATTAGCATCTGTTTCCAAATG

TTATCCCCCACTTAAGGGCAGGTTACCCACGTGTTACTCACCCGTCGCCACT

CGTTCAAAATTAAATCAAGATGCAAGCACCTTTCAATAATCAGAACTCGTT

CGACTTGCATGTAT.

3. Lactobacillus rhamnosus X253 bénéfique pour la santé buccale selon la revendication 1, **caractérisé en ce que** sa séquence génétique Phes est la suivante :

GGGGGAAAAATAGCCGCACGCAAACATTCGGATGCACCATGCCGGCACCG

AGAACTTCAATCCAGCCGGTATACTTGCAAACAGGGCAACCCTTGCCACCG

CAGCGGAAGCAGGAAACGTCCACTTCTACAGACGGTTCTGTAAACGGAAA

ATAGCTCGGGCGCAGGCGAATCGTCCGATCAGCGCCAAACACATGCTGAC

ACATGGCGAGTAACGTCCCCTTAAGATCAGCCATTGTAATATGCTTGTCGAT

CACCAGACCTTCCATCTGATGGAACTGGTGGCTATGTGTGGCATCATCATCA

TCACGCCGATAAACCACGCCCGGACTGATCATTTTCAGCGGGCCCTTAGTA

AAATCATGTTTCTCCATCGTCCGTGCCTGCATCGGGCTGGTTTGGGAACGCA

TCAGCAACTCATTGGTAATATAAAAGTTTCCTGCATATCACGAGCATGGAT

GACGCCGA.

EP 4 074 817 B1

4. Lactobacillus rhamnosus X253 selon l'une quelconque des revendications 1 à 3 pour l'utilisation dans la prévention et le traitement des maladies buccales et l'amélioration de la santé buccale.

5. Lactobacillus rhamnosus X253 pour l'utilisation selon la revendication 4, dans lequel ledit Lactobacillus rhamnosus X253 est inclus dans un produit destiné à améliorer la santé buccale, ledit produit étant choisi parmi le dentifrice, le bain de bouche, les cachets oraux probiotiques, une boisson au lactobacille et du lait fermenté probiotique.

Fig. 1

Fig. 2